# EUROPEAN PATENT SPECIFICATION

(11) **EP 3 227 803 B1**
(45) Date of publication and mention of the grant of the patent: **31.03.2021**
(21) Application number: 15804461.0
(22) Date of filing: 02.12.2015
(51) Int. Cl.: G16H 50/30

(54) **METHOD AND SYSTEM FOR PROVIDING CRITICAL CARE USING WEARABLE DEVICES**
VERFAHREN UND SYSTEM ZUR BEREITSTELLUNG VON INTENSIVPFLEGE MIT TRAGBAREN GERÄTEN
PROCEDE ET SYSTEME DE FOURNITURE DE SOINS CRITIQUES AU MOYEN DE DISPOSITIFS PORTABLES

(30) Priority: 03.12.2014 US 201462087082 P; 26.05.2015 EP 15169220
(43) Date of publication of application: 11.10.2017
(73) Proprietor: Koninklijke Philips N.V., 5656 AG Eindhoven (NL)
(72) Inventor: CRONIN, John, 5656 AE Eindhoven (NL); TEN KATE, Warner Rudolph Theophile, 5656 AE Eindhoven (NL)
(74) Representative: de Haan, Poul Erik
(86) International application number: PCT/EP2015/078396
(87) International publication number: WO 2016/087537

(56) References cited:
- US-A1- 2009 327 102
- US-A1- 2014 106 677
- US-A1- 2014 149 244
- US-B1- 8 359 288
- Ronnie D Caytiles ET AL: "u-Healthcare: The Next Healthcare Service Paradigm", International Journal of Bio-Science and Bio-Technology, Vol. 4, No. 2, 2 June 2012 (2012-06-02), pages 78-82, XP055228656, Retrieved from the Internet: URL:http://www.sersc.org/journals/IJBSBT/v ol4_no2/7.pdf [retrieved on 2015-11-16]
- BOULOS MAGED N KAMEL: "Location-based health information services: a new paradigm in personalised information delivery", INTERNATIONAL JOURNAL OF HEALTH GEOGRAPHICS, BIOMED CENTRAL LTD, LONDON, GB, vol. 2, no. 1, 10 January 2003 (2003-01-10), page 2, XP021018193, ISSN: 1476-072X, DOI: 10.1186/1476-072X-2-2
- STEFAN STEINIGER ET AL: "Foundations of Location Based Services", LECTURE NOTES ON LBS, , vol. 1.0 1 January 2006 (2006-01-01), pages 1-28, XP007908352, Retrieved from the Internet: URL:http://www.geo.unizh.ch/publications/c artouche/lbs_lecturenotes_steinigeretal200 6.pdf [retrieved on 2015-11-19]

## Description

### FIELD OF INVENTION

The present invention generally relates to wearable technology. More specifically, the present invention relates to systems and methods for providing critical care using wearable devices.

### BACKGROUND

Wearable technology is a new class of electronic systems that can provide data acquisition through a variety of unobtrusive sensors that may be worn by a user. The sensors gather information, for example, about the environment, the user's activity, or the user's health status. For instance, devices exist for tracking individual health parameters, such as heartbeat or walking steps. However, there are significant challenges related to the coordination, computation, communication, privacy, security, and presentation of the collected data.

Additionally, there are challenges related to power management given the current state of battery technology. Furthermore, analysis of the data is needed to make the data gathered by the sensors useful and relevant to end-users. In some cases, additional sources of information may be used to supplement the data gathered by the sensors. The many challenges that wearable technology presents require new designs in hardware and software.

There may be situations when individuals are in danger and a means of contacting emergency services may be desired. Such emergency services may include calling 911 or some form of security service. Alternatively, emergency and critical care asset maps (e.g., maps locating nearby hospitals) can typically be provided to inform an individual where some, but not all, important assets are located. However, knowing the location of the nearest asset, e.g., a hospital, does not always result in the timely healthcare service. For example, these maps may at times be out-of-date, which can cause a patient who is undergoing an emergency to waste time that could in some cases cost his/her life. Also, the time to travel to the nearest location of the hospital providing the needed care can be prohibitively long when the lives of the individuals are in imminent danger.

### SUMMARY OF THE INVENTION

It would be advantageous to have a system and method for providing a wearable device with critical care geofence abilities. The invention is based on understanding that a risk assessment for a user having a critical condition is a function of one or more health parameters of the user relevant to the critical condition and a number of assets in proximity to the user that can provide healthcare services addressing the critical condition of the user.

To better address this concern, a first aspect of the present invention includes a computer-implemented method for providing critical care using a wearable device. The method includes monitoring one or more health parameters related to a critical care condition of a user of a wearable device via one or more sensors in the wearable device; identifying one or more locations of assets relevant to the critical care condition; generating a risk assessment based on values of the monitored one or more health parameters and a number of the assets located within a radius from a current location of the wearable device; generating alert information to the user of the wearable device based on the risk assessment; and calling another device and/ or sending an SMS or email message to another device, wherein the calling and/ or the sending is based on the generated risk assessment, wherein at least some steps of the method are performed by a processor of the wearable device.

A further aspect of the invention provides a system for providing critical care using a wearable device, the system including: one or more critical care network servers, each server providing information regarding locations of assets, each asset designated as being relevant to one or more critical care conditions; and a wearable device comprising: memory configured to store information regarding a critical care condition associated with a user; one or more sensors configured to monitor one or more health parameters related to the critical care condition associated with the user; a processor configured for: identifying locations of assets determined to be relevant to the critical care condition associated with the user, wherein the locations are identified as being within a predetermined radius of a current location of the user; generating a risk assessment based on the monitored parameters and a number of the assets located within a radius from a current location of the wearable device; generating alert information to the user of the wearable device based on the risk assessment; and calling another device and/ or sending an SMS or email message to another device, wherein the calling and/ or the sending is based on the generated risk assessment; and a display screen configured to display the alert information.

A further aspect of the invention provides a non-transitory computer-readable storage medium, having embodied thereon a program executable by a processor to perform a method for providing on-demand wireless services, the method including: storing critical care information in a memory of the wearable device, the critical care information associated with a critical care condition of a user of the wearable device; monitoring one or more health parameters related to the critical care condition via one or more sensors in the wearable device; identifying one or more assets within a predetermined radius of a current location of the user, each asset being relevant to the critical care condition; generating a risk assessment based on the one or more health parameters and a number of the assets located within a radius from a current location of the wearable device; generating alert information to a user of the wearable device based on the risk assessment; and calling another device and/ or sending an SMS or email message to another device, wherein the calling and/ or the sending is based on the generated risk assessment.

The invention is defined by the appended claims.

### BRIEF DESCRIPTION OF THE DRAWINGS

FIG. 1 illustrates a diagram of different critical care issues and corresponding sensors in an exemplary wearable device with critical care capabilities.
FIG. 2 illustrates a network environment in which an exemplary system for providing a wearable device with critical care capabilities may be implemented according to an embodiment of the present invention.
FIG. 3A illustrates an exemplary geofence interface where the risk assessment indicates a low risk according to an embodiment of the present invention.
FIG. 3B illustrates an exemplary geofence interface where the risk assessment indicates a medium risk according to an embodiment of the present invention.
FIG. 3C illustrates an exemplary geofence interface where the risk assessment indicates a medium risk according to an embodiment of the present invention.
FIG. 3D illustrates an exemplary geofence interface where the risk assessment indicates a high risk according to an embodiment of the present invention.
FIG. 3E illustrates an exemplary geofence interface where the risk assessment indicates a high risk according to an embodiment of the present invention.
FIG. 4 illustrates an exemplary critical care graphical user interface (GUI) that may be used in a system for providing a wearable device with critical care capabilities according to an embodiment of the present invention.
FIG. 5 shows a flowchart illustrating an exemplary base software module for providing a wearable device with critical care capabilities according to an embodiment of the present invention.
FIG. 6 illustrates a mobile device architecture that may be utilized to implement the various features and processes described herein according to an embodiment of the present invention.
FIG. 7 shows a flowchart illustrating operation of an exemplary critical care software module for providing a wearable device with critical care capabilities according to an embodiment of the present invention.
FIG. 8 illustrates an exemplary critical care database that may be used in a system for providing a wearable device with critical care capabilities according to an embodiment of the present invention.
FIG. 9A shows a flowchart illustrating exemplary third party operations of an exemplary critical care software module of the critical care network according to an embodiment of the present invention.
FIG. 9B shows a flowchart illustrating exemplary user operations of an exemplary critical care software module of the critical care network according to an embodiment of the present invention.
FIG. 10 shows a flowchart illustrating an exemplary method for providing a wearable device with critical care capabilities according to an embodiment of the present invention.
FIG. 11 shows a block diagram of a computer-implemented method for providing critical care using a wearable device according to one embodiment of then invnetion.
FIG. 12 shows a block diagram of a method for determining a radius for generating a risk assessment according to some embodiments of the invention.

### DETAILED DESCRIPTION

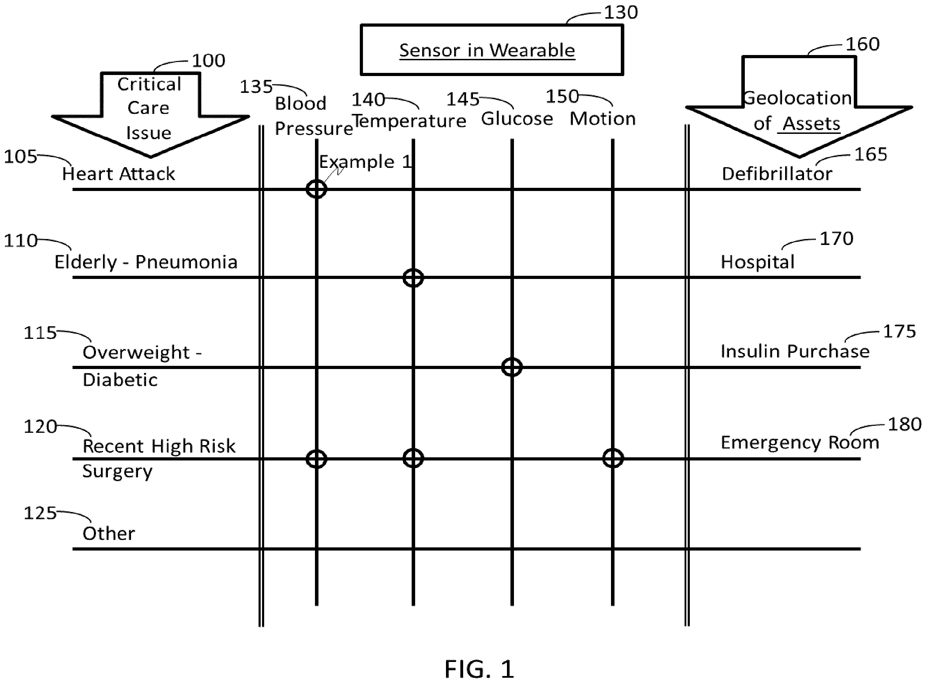

FIG. 1 illustrates a diagram of different critical care issues 100 and corresponding sensors 130 in an exemplary wearable device with critical care capabilities. Critical care issues 100 may be correlated with certain parameters detectable by sensors 130 in a wearable device, as well as with the geolocation of assets 160 for treating or otherwise responding to the respective issue. For example, an individual susceptible to heart attacks 105 may have their blood pressure 135 monitored by their wearable device. Additionally, that same individual may also have their wearable device identifying the locations of nearby defibrillators 165 in case a heart attack 105 occurs.

Other critical care issues may involve elderly individuals concerned about their temperature 140 (e.g., risk of pneumonia 110) and want to determine proximity to a hospital 170, a caregiver, or a shelter. Individuals who are overweight and/or diabetic 115 may be concerned about their glucose 145 and/or insulin levels and want to determine how far they may be from a place where insulin may be supplied or purchased 175. An individual who recently underwent a high-risk surgery 120 may have some health parameters monitored (e.g., blood pressure 135, temperature 140), motion parameters 150 monitored (e.g., in case of a fall), and may want to determine their distance from an emergency room 180. In each of the foregoing scenarios, a wearable device may be able to monitor an individual's parameters via specific sensors 130 to identify high-risk conditions as they develop in real-time, as well as provide follow-up assistance or information responsive to related emergency situations. More specifically, the wearable device may assist by providing geolocations of assets 160 (e.g., hospitals 170) to the user of the wearable device in response to a critical care issue 100 that may be impacted via a health parameter sensor measurement 130 from the wearable device. It should be understood that the critical care issues 100, the sensors 130, and the geolocation of assets 160 intended to be illustrative rather than limiting.

Some embodiments of an invention are based on understanding that a risk assessment for a user having a critical condition is a function of one or more health parameters of the user relevant to the critical condition and a number of assets in proximity to the user that can provide healthcare services addressing the critical condition of the user. For example, the risk assessment can be increased if the health parameters of the user are worsening, and can be increased if a number of relevant healthcare assets are not located nearby.

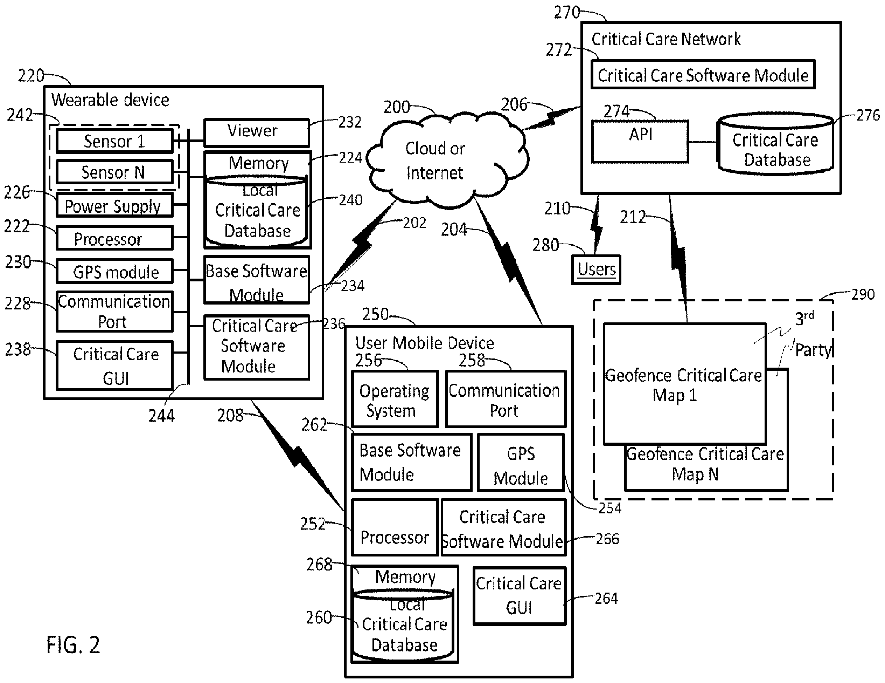

FIG. 2 illustrates a network environment in which an exemplary system for providing a wearable device 220 with critical care capabilities may be implemented. Such a network environment may include a wearable device 220, a user mobile device 250, a critical care network 270, a critical care database 276, and one or more third party geofencing map 290 related to critical care (e.g., geofence critical care maps maps 1-N 290). Each of the devices may communicate with each other directly (e.g., connections 208, 210, and 212) or via the cloud/Internet (e.g., connections 202, 204, and 206).

The wearable device may include a variety of components and software elements. These may include a viewer module 232, a memory 224 containing a local critical care database 240, a base software module 234, a critical care software module 236, a critical care graphic user interface (GUI) 238, a wired and/or wireless communication port/module 228 (e.g, a USB port module, a FireWire port module, a Lightning port module, a Thunderbolt port module, a Wi-Fi connection module, a 3G/4G/LTE cellular connection module, a Bluetooth connection module, a Bluetooth low energy connection module, a , Bluetooth Smart connection module, a near field communication module, a radio wave communications module), a global positioning system (GPS) module 230, a processor 222, a power supply 226 (e.g., a rechargeable or non-rechargeable battery), and one or more sensors 242 (i.e., sensors 1-N 242). In one embodiment, these components or modules may be connected via a single bus 244, while in other embodiments, the wearable device may have more of a divided architecture. It should be understood that the components or modules of wearable device 220 as illustrated in FIG. 2 and described above are illustrative rather than limiting. A wearable device 220 need not include all of these components and/or may include additional components not listed herein.

The mobile user device, which may connect directly with the wearable device (i.e., connection 208), may also include a variety of components and software elements. These may include a mobile user device's own operating system (OS) 256, a memory 268, a GPS module 254, a base software module 262, a critical care software module 266, a critical care GUI 264, a processor 252, and a wired and/or wireless communication port/module 258 (e.g, a USB port module, a FireWire port module, a Lightning port module, a Thunderbolt port module, a Wi-Fi connection module, a 3G/4G/LTE cellular connection module, a Bluetooth connection module, a Bluetooth low energy connection module, a, Bluetooth Smart connection module, a near field communication module, a radio wave communications module). The mobile user device may, in some embodiments, include a different set of capabilities than the wearable device (*e.g*., different types of input/output (I/O), communication links to the cloud/Internet, and/or display). The specific components of the mobile user device can be dependent on the quality and design of the mobile user device. In one embodiment, the user mobile device 250 may connect directly to the wearable device 220 through a connection 208. These connections may be performed through communication port/module 258. In one embodiment, the elements of user mobile device 250 may communicate with each other using a single communication bus, while in other embodiments, the user device may have more of a divided architecture. It should be understood that the components and software elements of user mobile device 250 as illustrated in FIG. 2 and described above are illustrative rather than limiting. A user mobile device 250 need not include all of these listed components and/or may include additional components not listed herein.

In one embodiment, the user mobile device 250 may be, for example, a smartphone device, a tablet device, a laptop computer, a desktop computer, a portable gaming console, a home gaming console, a smart television, a home entertainment system, an automobile dashboard computer, a watercraft computer, an aircraft computer, a second wearable device, or another type of device with computing capabilities.

The critical care network 270 may include one or more servers, which may store (individually or in a distributed fashion) critical care software module 272 and a third party geofencing map database a critical care database 276 in memory, as well as an application programming interface (API) 274 that can access or otherwise provide users 280 with geofence critical care maps maps 290. The geofence critical care maps maps 290 (*e.g.,* maps of where defibrillators 165 are located) may be loaded into the critical care database 276 through the API 274. The critical care software module 272 may pull the information from the API 274, set up the critical care database 276, and provide a critical care database 276 for a particular functionality. In some embodiments, multiple critical care databases 276 may be created by the critical care network 270, and organized by user 280, by critical care issue 100 *(e.g.,* diabetes 115), by asset 160 *(e.g.,* defibrillators), by sensors 242 *(e.g.,* blood pressure), or by some others. The critical care database 276 may store map data regarding specific critical care issues (*e.g.,* regarding particular health risks, what asset may be tied to the particular health risk, where the asset can be found). In some embodiments, users 280 may access the API 274 and other portions of the critical care network 270 using a connection 210, which may be a connection running through the cloud/internet 200 or may be a direct connection. Likewise, the critical care network 270 may access the geofence critical care maps through a connection 212, which may be a connection running through the cloud/internet 200 or may be a direct connection (*e.g.,* geofence critical care maps 1-N 290 may be cached locally at the critical care network 270 and occasionally updated through an internet/cloud 200 connection 206).

The wearable device 220 may allow a user to access a critical care GUI 238. In the critical care GUI 238, the user may be able to input information including their critical care issue 100 *(e.g.,* heart attack risk 105), select sensors 242 to be used to monitor parameters desired *(e.g.,* blood pressure 135), locate desired assets *(e.g.,* defibrillators 165), and provide a set of rules concerning parameters (*e.g.,* detect when blood pressure is higher than a threshold, inform the user when their blood pressure meets the high threshold) or rules about accessibility for assets (*e.g.,* there should be 'x' number of defibrillators 165 within a two mile geofence).

A geofence is a tool that uses GPS (which may be interfaced with using the wearable device GPS module 230 or the mobile user device GPS module 254) or radio frequency identification (RFID) (which may be interfaced with using the wearable device's communications port/module 228 or the mobile user device's communications port/module 258) to define a geographical boundary. Referring to the example provided above regarding a need for 'x' number of defibrillators 165 to be present within a two mile geofence, the GPS or RFID may use one or more geofence critical care maps maps 290 to identify all available defibrillators 165 within a two mile radius from the user (whose location may be given by the wearable device's GPS module 230 or the mobile user device's GPS module 254). If such condition cannot be satisfied, a notification may be provided to inform the user (or designated health professional) when a particular area does not meet the rule, thereby suggesting that such an area may not be a desirable one to enter or stay. Such a notification may be provided using the wearable device 220 or the user mobile device 250. In this way, the geofence may operate as a virtual barrier notifying the user when he/she is leaving a "safe" zone with nearby assets (*e.g.,* defibrillators 165) and entering an "unsafe" zone without nearby assets (*e.g.,* defibrillators 165).

For example, some embodiments determine a radius defining the geofence based on time required to access the relevant asset. For example, one embodiment determines a maximal period of time for accessing the asset based on a type of the critical care condition. For example a maximal time required to access a defibrillator can be less than a maximal time required to receive an insulin injection. Also, one embodiment automatically detects the current transportation used by the user, and determines the radius as a maximal distance of traveling for the maximal period of time using the detected transportation. Examples of the transportation include traveling by a car and traveling by walking.

After the information is provided by the user, the base software module 234 may be polled at regular intervals (*e.g.,* every ten minutes). The critical care software module 236 may also go to the local critical care database 240 and use the information stored there. It should be noted that the local critical care database 240 may be synchronized with the critical care database 276 found in the critical care network 270 and/or with the local critical care database 260 found in the user mobile device 250. Such synchronization may be performed periodically, continuously, or upon a triggering event (*e.g.,* a user interaction using the critical care GUI 238 of the wearable device 220, a user interaction using the critical care GUI 264 of the user mobile device 250, a trigger in the critical care software module 272 of the critical care network, an updating of one of the geofence critical care maps 290, an updating of any one of the critical care databases 240/260/276, a reboot of the wearable device 220 or user mobile device 250, a new connection to the cloud/internet 200 after a temporary connection loss, or a movement of the user as determined by the GPS module 230 of the wearable device 220 or by the GPS module 254 of the user mobile device 250).

Furthermore, if the geolocation changes (*e.g.,* the user moves to a new location as determined by the GPS module 230 of the wearable device 220 or by the GPS module 254 of the user mobile device 250), the critical care software module 272 may then provide such information to one or more servers of the critical care network 270. For example, the wearable device and/or mobile device of the user may upload the information provided via the critical care GUI 238 of the wearable device 220 or the critical care GUI 264 of the user mobile device 250. In response, the critical care network 270 may provide information as to the number of assets 160 available, the availability of assets 160 within a geofence, and details about potential issues arising from one or more health parameters being monitored (*e.g.,* blood pressure). In return, the user may be able to obtain a risk assessment viewable on a display (*e.g.,* the viewer module 232 on the wearable device 220 or a display of the user mobile device 250). Such risk assessment may take into account the risk of a critical care condition 100 (*e.g.,* high blood pressure) and available assets 160 for that condition within the geofence (*e.g.,* a hospital 170).

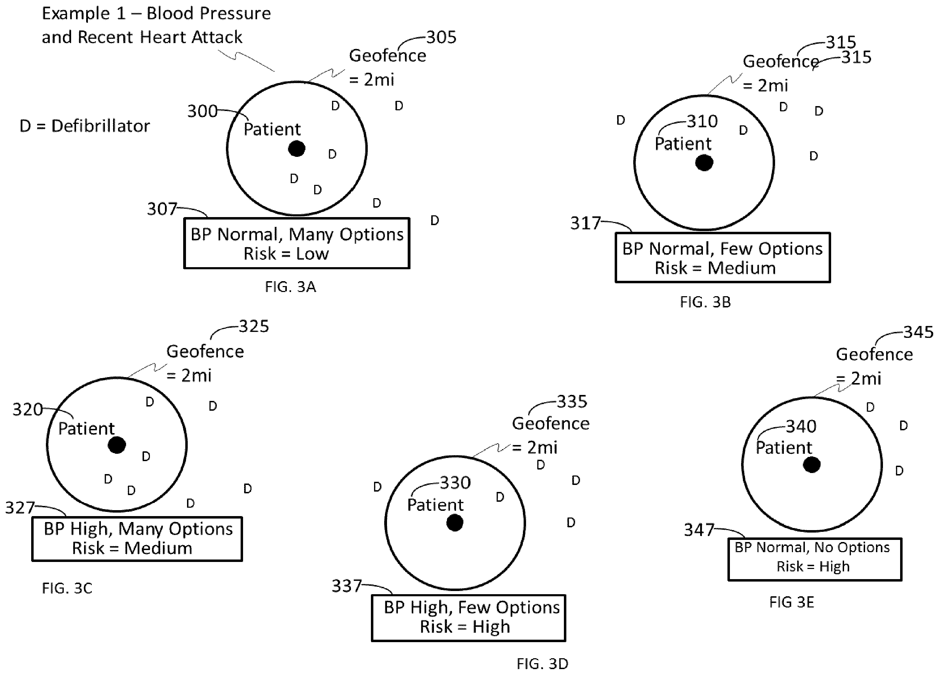

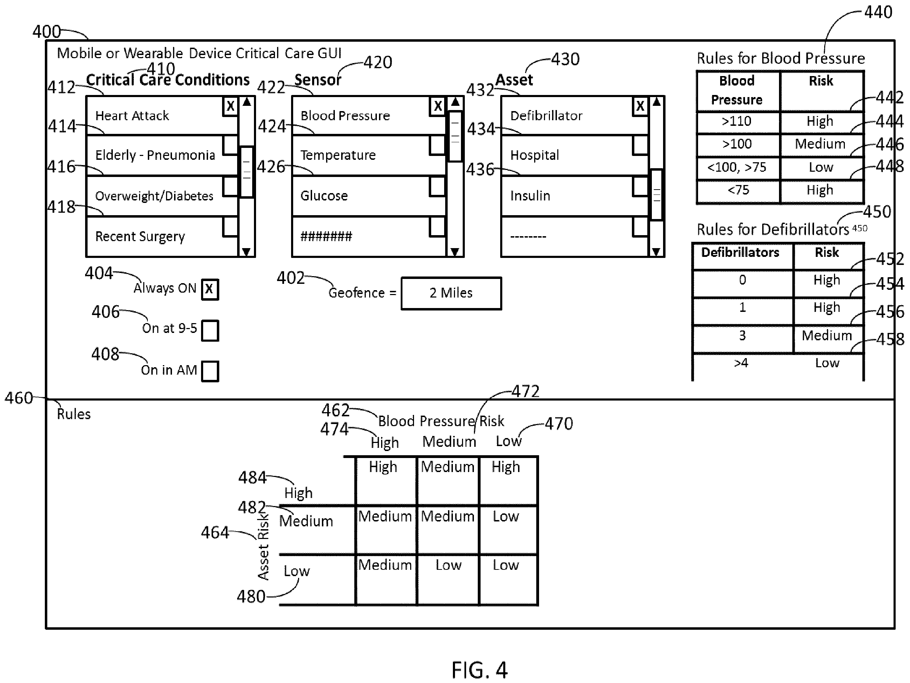

FIGS. 3A-E illustrate different examples of geolocation that may be used in a system for providing a wearable device with critical care capabilities. A geofence can be designated as being a two mile radius from the user. Asset availability may be identified in different manners toward the user. In some embodiments, the user could simply receive a number of assets (*e.g.,* "3 hospitals within 2 miles"), or a list of latitude/longitude coordinates or street intersections at which assets can be found. In the embodiments illustrated in FIGS. 3A-E, asset availability may be identified using a visual geofence "scanner" graphic showing asset locations (*e.g.,* available defibrillators designated by the letter "D") within each geofence around the user. The two mile radius can be altered based on the user's preference through the critical care GUI 238 of the wearable device 220 or the critical care GUI 264 of the user mobile device 250 (*e.g.,* geofence setting 402 in FIG. 4).

FIG. 3A illustrates an exemplary geofence interface where the risk assessment 307 indicates a low risk. The low risk in FIG. 3A is based on the blood pressure of the patient 300 being determined to be presently normal, and the presence of many available assets (*e.g.,* defibrillators "D") within the geofence 305.

FIG. 3B illustrates an exemplary geofence interface where the risk assessment 317 indicates a medium risk. The medium risk in FIG. 3B is based on the blood pressure of the patient 310 being determined to be presently normal, and the presence of few available assets (*e.g.,* defibrillators "D") within the geofence 315.

FIG. 3C illustrates an exemplary geofence interface where the risk assessment 327 indicates a medium risk. The medium risk in FIG. 3C is based on the blood pressure of the patient 320 being determined to be presently high, and the presence of many available assets (*e.g.,* defibrillators "D") within the geofence 325.

FIG. 3D illustrates an exemplary geofence interface where the risk assessment 337 indicates a high risk. The high risk in FIG. 3D is based on the blood pressure of the patient 330 being determined to be presently high, and the presence of few available assets (*e.g.,* defibrillators "D") within the geofence 335.

FIG. 3E illustrates an exemplary geofence interface where the risk assessment 347 indicates a high risk. The high risk in FIG. 3E is based on the blood pressure of the patient 340 being determined to be presently normal, and the presence of no available assets (*e.g.,* no defibrillators "D") within the geofence 345.

The determination as to what may constitute as low, medium, or high risk could be provided through a set of algorithms and/or rules, which may be modified as desired by the user or designated administrator. In one embodiment, this determination may be adjusted via the critical care GUI 238 of the wearable device 220 or the critical care GUI 264 of the user mobile device 250 (*e.g.,* rules interfaces 440, 450, and 460 in FIG. 4).

FIG. 4 illustrates an exemplary critical care graphical user interface (GUI) 400 that may be used in a system for providing a wearable device 220 with critical care capabilities. The exemplary critical care GUI 400 may be an embodiment of the critical care GUI 238 of the wearable device 220 or an embodiment of the critical care GUI 264 of the user mobile device 250. The exemplary critical care GUI 400 may include a drop-down selection or scrolling menu of various critical care conditions 410, including heart attack 412, pneumonia for an elderly individual 414, overweight (or diabetes) 416, and recent surgery 418. There may be other critical care conditions of interest that may also be included in this drop-down selection or scrolling menu as well. As illustrated, in this embodiment, a critical care condition 410 (*e.g.,* heart attack 412) may be selected in the drop-down selection or scrolling menu and indicated by an 'x' in the box. In other embodiments, a different selection mechanism can be employed, such a visual grouping of icons representing critical care issues, or radio buttons, or checkmarks, or interfaces allowing a user to circle an appropriate critical care condition 410. By selecting a particular critical care condition 410, a particular health parameter (*e.g.,* heart rate 412) could be designated as a primary factor to monitor.

The critical care condition 410 selected may influence the type of sensors 420 selected (or, in some embodiments, made selectable) within the critical care GUI 400, as illustrated in the next drop-down selection or scrolling menu, to provide the types of sensors available 420. For example, the available sensors 420 could monitor blood pressure 422, temperature 424, glucose 426, or another health parameter. In this case, the user may select the blood pressure sensor 420 in much the same way as when selecting the critical care condition 410.

The user may also be able to select the type of desired asset 430. The user asset selection may be influenced by his/her critical care condition 410 selected and/or his/her sensor 420 selected. In some embodiments, the critical care GUI 400 may limit the assets 430 available for selection based on the user's selections regarding their critical care condition 310 and sensors 420 (*e.g.,* the "insulin provider/seller" asset 436 could be withheld unless the user has selected the "overweight/diabetes" critical care condition 416 and/or the "glucose" sensor 426). The selectable assets 430 may include defibrillators 432 (*e.g.,* for heart attack condition 412), hospital 434 (*e.g.,* for elderly users 414 or recent surgery users 418), or insulin 436 (*e.g.,* for diabetic individuals 416).

The critical care GUI 400 may also include a "rules " input area (440, 450, 460) where the user could provide rules or guidelines so that the wearable device and/or the user mobile device can quantify particular health conditions based on the sensor data obtained. For example, the user could use a "rules for blood pressure" interface 440 to define what thresholds for blood pressure are considered low risk 446 (*e.g.,* between 75 and 100 mm Hg), medium risk 444 (*e.g.,* between 100 and 110 mm Hg), high risk due to high blood pressure 442 (*e.g.,* over 110 mm Hg), or high risk due to low blood pressure 448 (*e.g.,* less than 75 mm Hg). Similarly, the user could use a "rules for defibrillators" interface 450 to define what thresholds for blood pressure are.

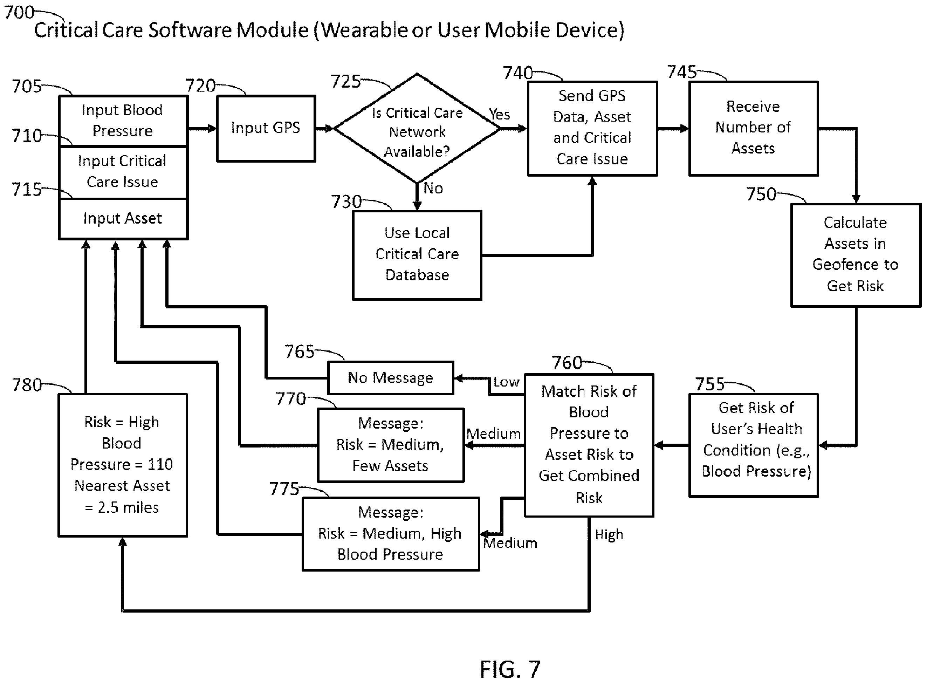

Furthermore, the user can also provide a risk allocation based on a likelihood of availability of assets within a geofence. For example, the user could use a "rules for defibrillators" interface 450 to define what level of risk is associated with what number of defibrillators in the user's geofence area. For example, a user could define a low asset risk 458 (*e.g.,* more than four defibrillators within the geofence area), a medium asset risk 456 (*e.g.,* two or three defibrillators within the geofence area), or a high asset risk 454 or 452 (*e.g.,* one or no defibrillators within the geofence area). In some embodiments, a rule set for an asset such as a defibrillator could also take into account how many of the asset items are in use or might be used by other nearby users (*e.g.,* how many defibrilators). The combination and weight of the two sets of rules (*e.g.,* sensor rules for blood pressure 440 and rules for the number of assets within an area 450) may provide an appropriate approximation of the user's risk assessment. In other words, the exemplary critical care GUI 400, as displayed on the user mobile device 250 and/or the wearable device 220, may include a combined rules interface 460, wherein the user can define how an estimate is calculated as to the user's safety based on the user's present condition (as selected in 410), sensor readings (as selected in 420), and availability of assets (as selected in 430) to resolve that condition if that condition worsen. For example, the combined rules interface 460 of FIG. 4 plots a grid, with blood pressure risk 462 along a horizontal axis and asset risk 464 along a vertical axis. Rules could then be input via a user selecting what type of risk should be estimated (which affects actions taken by the wearable device 220 or user mobile device 250) given a high blood pressure risk 474, a medium blood pressure risk 472, and a low blood pressure risk 470, as combined with a high asset risk 484, a medium asset risk 482, and a low asset risk 480. For example, a high asset risk 484 combined with a medium blood pressure risk 472 may result in a medium overall risk estimate. In one embodiment, a predefined look-up table stored in a critical care database, either locally or in one of the servers of the critical care network, may be used for determining the type of action corresponds to a combination of health condition risk (e.g., blood pressure at high risk) and asset risk (e.g., number of defibrillators within a preset radius). The predefined look-up table is an array of data containing all the possible combinations of health condition risk and asset risk that match the corresponding one or more types of action, which are further discussed in detail in FIG. 7.

The exemplary critical care GUI 400 may also include other settings. For example, the user can adjust the size of his/her geofence using the geofence setting 402 (*e.g.,* 2 miles). The user could also adjust whether the critical care software module 236 of the wearable device and/or the critical care software module 266 of the user mobile device are always on 404 (*e.g.,* yes), whether they are on during the 9:00-5:00 hours 406 (*e.g.,* no), and whether they are on in the morning 408 (*e.g.,* no). It should be understood that the exemplary critical care GUI of FIG. 4 is intended to be illustrative rather than limiting; in some embodiments, more/different settings and interfaces may be included, while in other embodiments, settings and interfaces may be missing that are present in FIG. 4.

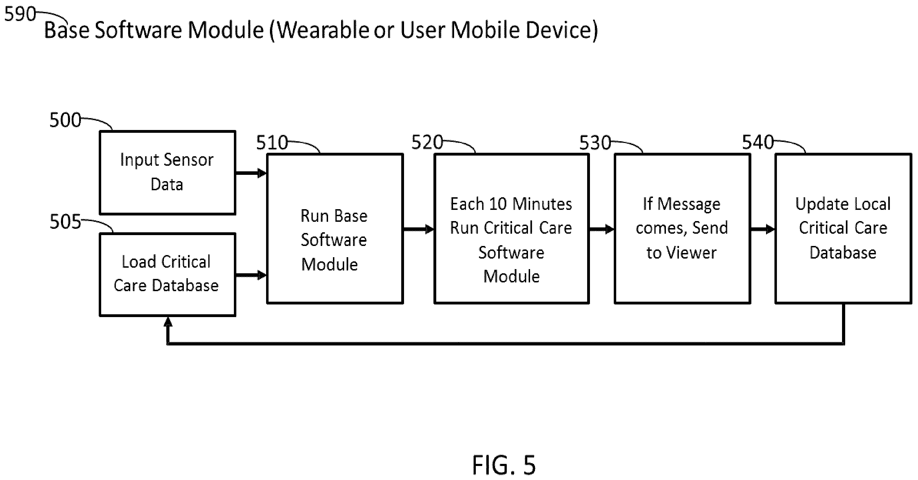

FIG. 5 is a flowchart illustrating an exemplary base software module 590 for providing a wearable device 220 with critical care capabilities. The exemplary base software module 590 may be an embodiment of the base software module 234 of the wearable device 220, or it may be an embodiment of the base software module 262 of the user mobile device 250.

According to one embodiment, sensor data (step 500) and a critical care database (505) may be provided (*e.g.,* through the Internet 200 or through a direct connection such as connection 208) to designated devices running the exemplary base software module 590 (step 510). At regular intervals of time (*e.g.,* ten minutes), the devices may run the critical care software module (*e.g.* critical care software module 236 of the wearable device module 220 and/or critical care software module 266 of the user mobile device 250) (step 520). In certain situations (*e.g.,* a medium risk or high risk situation is present), an action (*e.g.,* a notification or alert message, graphic, video, sound, vibration, or small electric shock) is performed. In situations where a notification or alert message is triggered (*e.g.,* a warning message regarding a negative condition like high blood pressure), the message may be provided to the display/viewer (*e.g.,* viewer 232 of the wearable device 220 or a display of the user mobile device 250) so that the user can view the message (step 530). The devices may also update their local critical care databases (*e.g.,* the local critical care database 240 of the wearable device 220 and/or the local critical care database 260 of the user mobile device 250) (step 540). The updated local critical care database(s) may then be loaded (step 505) and fed back into the base software module (step 510). In some embodiments, the local critical care databases (*e.g.,* the local critical care database 240 of the wearable device 220 and/or the local critical care database 260 of the user mobile device 250) may also be synchronized with the critical care database 276 of the critical care network 270.

While the flow diagram in FIG. 5 shows a particular order of operations performed by certain embodiments of the invention, it should be understood that such order is exemplary (e.g., alternative embodiments can perform the operations in a different order, combine certain operations, overlap certain operations, etc.).

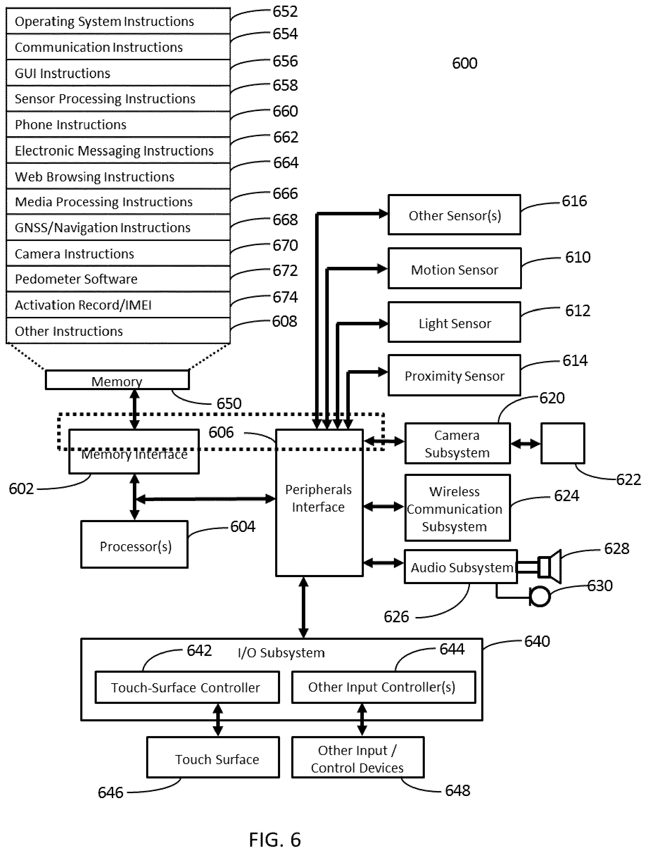

FIG. 6 illustrates an exemplary computing device architecture that may be utilized to implement the various features and processes described herein. For example, the computing device architecture 600 could be implemented in a pedometer. Architecture 600 as illustrated in FIG. 6 includes memory interface 602, processors 604, and peripheral interface 606. Memory interface 602, processors 604 and peripherals interface 606 can be separate components or can be integrated as a part of one or more integrated circuits. The various components can be coupled by one or more communication buses or signal lines.

Processors 604 as illustrated in FIG. 6 is meant to be inclusive of data processors, image processors, central processing unit, or any variety of multi-core processing devices. Any variety of sensors, external devices, and external subsystems can be coupled to peripherals interface 606 to facilitate any number of functionalities within the architecture 600 of the exemplar mobile device. For example, motion sensor 610, light sensor 612, and proximity sensor 614 can be coupled to peripherals interface 606 to facilitate orientation, lighting, and proximity functions of the mobile device. For example, light sensor 612 could be utilized to facilitate adjusting the brightness of touch surface 646. Motion sensor 610, which could be exemplified in the context of an accelerometer or gyroscope, could be utilized to detect movement and orientation of the mobile device. Display objects or media could then be presented according to a detected orientation (e.g., portrait or landscape).

Other sensors could be coupled to peripherals interface 606, such as a temperature sensor, a biometric sensor, or other sensing device to facilitate corresponding functionalities. Location processor 615 (e.g., a global positioning transceiver) can be coupled to peripherals interface 606 to allow for generation of geo-location data thereby facilitating geo-positioning. An electronic magnetometer 616 such as an integrated circuit chip could in turn be connected to peripherals interface 606 to provide data related to the direction of true magnetic North whereby the mobile device could enjoy compass or directional functionality. Camera subsystem 620 and an optical sensor 622 such as a charged coupled device (CCD) or a complementary metal-oxide semiconductor (CMOS) optical sensor can facilitate camera functions such as recording photographs and video clips.

Communication functionality can be facilitated through one or more communication subsystems 624, which may include one or more wireless communication subsystems. Wireless communication subsystems 624 can include 802.x or Bluetooth transceivers as well as optical transceivers such as infrared. Wired communication system can include a port device such as a Universal Serial Bus (USB) port or some other wired port connection that can be used to establish a wired coupling to other computing devices such as network access devices, personal computers, printers, displays, or other processing devices capable of receiving or transmitting data. The specific design and implementation of communication subsystem 624 may depend on the communication network or medium over which the device is intended to operate. For example, a device may include wireless communication subsystem designed to operate over a global system for mobile communications (GSM) network, a GPRS network, an enhanced data GSM environment (EDGE) network, 802.x communication networks, code division multiple access (CDMA) networks, or Bluetooth networks. Communication subsystem 624 may include hosting protocols such that the device may be configured as a base station for other wireless devices. Communication subsystems can also allow the device to synchronize with a host device using one or more protocols such as TCP/IP, HTTP, or UDP.

Audio subsystem 626 can be coupled to a speaker 628 and one or more microphones 630 to facilitate voice-enabled functions. These functions might include voice recognition, voice replication, or digital recording. Audio subsystem 626 in conjunction may also encompass traditional telephony functions.

I/O subsystem 640 may include touch controller 642 and/or other input controller(s) 644. Touch controller 642 can be coupled to a touch surface 646. Touch surface 646 and touch controller 642 may detect contact and movement or break thereof using any of a number of touch sensitivity technologies, including but not limited to capacitive, resistive, infrared, or surface acoustic wave technologies. Other proximity sensor arrays or elements for determining one or more points of contact with touch surface 646 may likewise be utilized. In one implementation, touch surface 646 can display virtual or soft buttons and a virtual keyboard, which can be used as an input/output device by the user.

Other input controllers 644 can be coupled to other input/control devices 648 such as one or more buttons, rocker switches, thumb-wheels, infrared ports, USB ports, and/or a pointer device such as a stylus. The one or more buttons (not shown) can include an up/down button for volume control of speaker 628 and/or microphone 630. In some implementations, device 600 can include the functionality of an audio and/or video playback or recording device and may include a pin connector for tethering to other devices.

Memory interface 602 can be coupled to memory 650. Memory 650 can include high-speed random access memory or non-volatile memory such as magnetic disk storage devices, optical storage devices, or flash memory. Memory 650 can store operating system 652, such as Darwin, RTXC, LINUX, UNIX, OS X, ANDROID, WINDOWS, or an embedded operating system such as VxWorks. Operating system 652 may include instructions for handling basic system services and for performing hardware dependent tasks. In some implementations, operating system 652 can include a kernel.

Memory 650 may also store communication instructions 654 to facilitate communicating with other mobile computing devices or servers. Communication instructions 654 can also be used to select an operational mode or communication medium for use by the device based on a geographic location, which could be obtained by the GPS/Navigation instructions 668. Memory 650 may include graphical user interface instructions 656 to facilitate graphic user interface processing such as the generation of an interface; sensor processing instructions 658 to facilitate sensor-related processing and functions; phone instructions 660 to facilitate phone-related processes and functions; electronic messaging instructions 662 to facilitate electronic-messaging related processes and functions; web browsing instructions 664 to facilitate web browsing-related processes and functions; media processing instructions 666 to facilitate media processing-related processes and functions; GPS/Navigation instructions 668 to facilitate GPS and navigation-related processes, camera instructions 670 to facilitate camera-related processes and functions; and other instructions 608 for any other application that may be operating on or in conjunction with the mobile computing device. Memory 650 may also store other software instructions for facilitating other processes, features and applications, such as applications related to navigation, social networking, location-based services or map displays.

Various embodiments described herein achieve various functionality through the execution of instructions by a processor. It will be understood that, while various examples are described in the context of instructions actively performing steps or other actions, any such actions will actually be performed by the processor that executes such instructions.

Note that the base software module (234, 262), the critical care software module (236, 266, 272), pedometer software 672, and activation record software 674 are softwares that are stored in one of the memory for execution by the processor (222, 252).

The memory (224, 268) may store operating system instructions 652, communication instructions 654, GUI instructions 656, sensor processing instructions 658, phone instructions 660, electronic messaging instructions 662, web browsing instructions 664, media processing instructions 666, GNSS/navigation instructions 668, camera instructions 670, and other instructions 608 for execution by the processor (222, 252). It will be understood that these instructions may be alternatively or additionally stored in a non-volatile storage device such as the storage device storing the reference link database or another storage device (not shown). For example, the instructions may be stored in a flash memory or an electronic read only memory (ROM) until they are to be executed by the processor, at which point they are copied to the memory (224, 268). As used herein, the term storage will be understood to refer to non-volatile memories.

The processor (222, 252) may be virtually any device capable of performing the functions described herein including the functions described above in connection with the operating system instructions 652, communication instructions 654, GUI instructions 656, sensor processing instructions 658, phone instructions 660, electronic messaging instructions 662, web browsing instructions 664, media processing instructions 666, GNSS/navigation instructions 668, camera instructions 670, and other instructions 608. For example, the processor (222, 252) may include one or more microprocessors, one or more field-programmable gate arrays (FPGA), or one or more application-specific integrated circuits (ASIC). In some embodiments, the processor may not utilize stored instructions to perform some or all of the functions described herein; for example, an ASIC may be hardwired to perform one or more of the functions describe above with reference to the operating system instructions 652, communication instructions 654, GUI instructions 656, sensor processing instructions 658, phone instructions 660, electronic messaging instructions 662, web browsing instructions 664, media processing instructions 666, GNSS/navigation instructions 668, camera instructions 670, and other instructions 608. In some such embodiments, the operating system instructions 652, communication instructions 654, GUI instructions 656, sensor processing instructions 658, phone instructions 660, electronic messaging instructions 662, web browsing instructions 664, media processing instructions 666, GNSS/navigation instructions 668, camera instructions 670, and other instructions 608 may be omitted because they are already embodied in the processor (222, 252) without the need for stored instructions.

Each of the above identified instructions and applications can correspond to a set of instructions for performing one or more functions described above. These instructions need not be implemented as separate software programs, procedures, or modules. Memory 650 can include additional or fewer instructions. Furthermore, various functions of the mobile device may be implemented in hardware and/or in software, including in one or more signal processing and/or application specific integrated circuits.

Certain features may be implemented in a computer system that includes a back-end component, such as a data server, that includes a middleware component, such as an application server or an Internet server, or that includes a front-end component, such as a client computer having a graphical user interface or an Internet browser, or any combination of the foregoing. The components of the system can be connected by any form or medium of digital data communication such as a communication network. Some examples of communication networks include LAN, WAN and the computers and networks forming the Internet. The computer system can include clients and servers. A client and server are generally remote from each other and typically interact through a network. The relationship of client and server arises by virtue of computer programs running on the respective computers and having a client-server relationship to each other.

One or more features or steps of the disclosed embodiments may be implemented using an API that can define on or more parameters that are passed between a calling application and other software code such as an operating system, library routine, function that provides a service, that provides data, or that performs an operation or a computation. The API can be implemented as one or more calls in program code that send or receive one or more parameters through a parameter list or other structure based on a call convention defined in an API specification document. A parameter can be a constant, a key, a data structure, an object, an object class, a variable, a data type, a pointer, an array, a list, or another call. API calls and parameters can be implemented in any programming language. The programming language can define the vocabulary and calling convention that a programmer will employ to access functions supporting the API. In some implementations, an API call can report to an application the capabilities of a device running the application, such as input capability, output capability, processing capability, power capability, and communications capability.

FIG. 7 is a flowchart illustrating an exemplary operation of an exemplary critical care software module 700 for providing a wearable device with critical care capabilities. The exemplary critical care software module 700 may be the critical care software module 236 of the wearable device 220, and/or it may be the critical care software module 266 of the user mobile device 250. The user may provide certain inputs (*e.g.,* the user's blood pressure 705, the user's critical care issue/condition 710, and the user's chosen geofence asset 715), as well as allow for location to be identified and provided through GPS (step 720).

After receiving the inputs (705, 710, 715, 720) from the user (*e.g.,* through the critical care GUI 238 or 264), the critical care software module 700 may then check to see if the critical care network 270 is available to be accessed (step 725). In situations where the network is not reachable, the critical care software module may use the local critical care database of the corresponding devices (*e.g.,* the local critical care database 240 of the wearable device 220 and/or the local critical care database 260 of the user mobile device 250) (step 730). Otherwise, if the network is available, the critical care software module 700 may provide the user's location (*e.g.,* GPS data) and the other inputted data (*e.g.,* a particular asset or critical care issue to be monitored) (step 740). From the information provided to the critical care network, the critical care software module 700 may receive a number corresponding to the number of assets available within a desired geofence (step 745), which in turn can be used to calculate and dictate an asset risk level based on the asset availability within the geofence (step 750).

The critical care software module 700 can also determine a sensor risk based on the particular sensor and/or critical care issue (*e.g.,* blood pressure) derived from input of the user's health conditions (step 755). With the risk associated with the particular critical care condition and the risk associated with the asset availability within the geofence, the critical care software module 700 can then determine a combined risk assessment (step 760), as discussed above with respect to FIGS. 3A-3E and combined rule interface 460 of FIG. 4.

According to the value of the risk assessment, different actions may be taken. In one embodiment, these may be different messages. For example, if the combined risk is determined to be low, the rule may dictate that no message is sent (step 765). If the combined risk is determined to be medium, a message warning the user of a medium risk could be issued, notifying the user why there was a medium risk, such as "few assets in the geofence area" (step 770) or "high blood pressure" (step 775). If the combined risk is determined to be high, a message warning the user of a high risk could be issued, notifying the user of more specific reasons for the high risk assessment (*e.g.,* high blood pressure at 100 mm Hg and nearest asset 2.5 miles away) (step 780). Action types are alerting the user with a vibration, alerting the user by displaying a graphic, alerting the user by playing a video clip, alerting the user by playing an audio clip, alerting the user by delivering a small electric shock. Furthermore, another device or individual (*e.g*., a doctor, a critical care professional, a caregiver, an emergency contact, or a family member is called or an SMS or e-mail message to another device or individual (*e.g*., a doctor, a critical care professional, a caregiver, an emergency contact, or a family is sent or a message from another device or individual (*e.g*., receiving a customized message from a critical care facility warning the user that the he/she has strayed far away from any geofence assets) is received. While the flow diagram in FIG. 7 shows a particular order of operations performed by certain embodiments of the invention, it should be understood that such order is exemplary (e.g., alternative embodiments can perform the operations in a different order, combine certain operations, overlap certain operations, etc.).

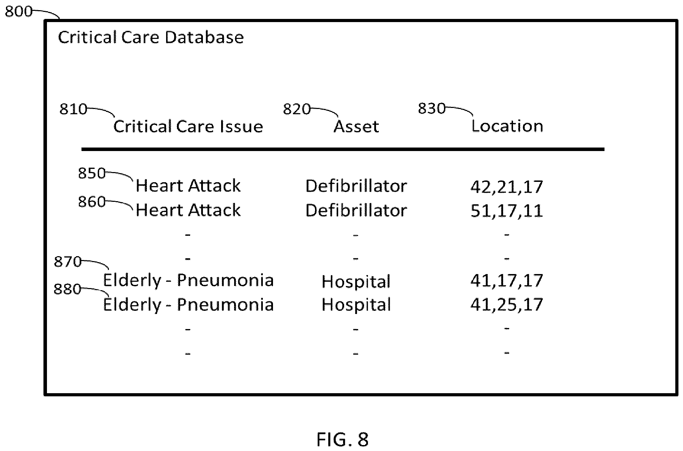

FIG. 8 illustrates an exemplary critical care database 800 that may be used in a system for providing a wearable device with critical care capabilities. The exemplary critical care database 800 may be an embodiment of the local critical care database 240 of the wearable device 220 and/or an embodiment of the local critical care database 260 of the user mobile device 250 and/or an embodiment of the critical care database 276 of the critical care network 270. The exemplary critical care database 800 may include data concerning critical care issues 810, types of geofence assets tracked 820, and the location of such assets 830. For example, in row 850 and row 860, where heart attack is the critical condition of interest (column 810), locations (column 830) of any number of different and available defibrillators (column 820) could be identified and provided. Based on the location of the different assets, the mobile and/or wearable device may be able to calculate how many of these assets may be found within a given geofence.

The exemplary critical care database 800 could provide the assets for the corresponding number of critical care issues for any number of assets and/or critical care issues. For example, in row 870 and row 880, the locations (column 830) of hospitals (column 820) for elderly individuals with pneumonia (column 810) are provided.

In some embodiments data may periodically be synchronized between the local critical care database 240 of the wearable device 220 and/or an embodiment of the local critical care database 260 of the user mobile device 250 and/or an embodiment of the critical care database 276 of the critical care network 270. For example, these may be synchronized after a user inputs data into one a local critical care database (*i.e.,* 240 or 260) through a critical care GUI (*i.e.,* 238 or 264).

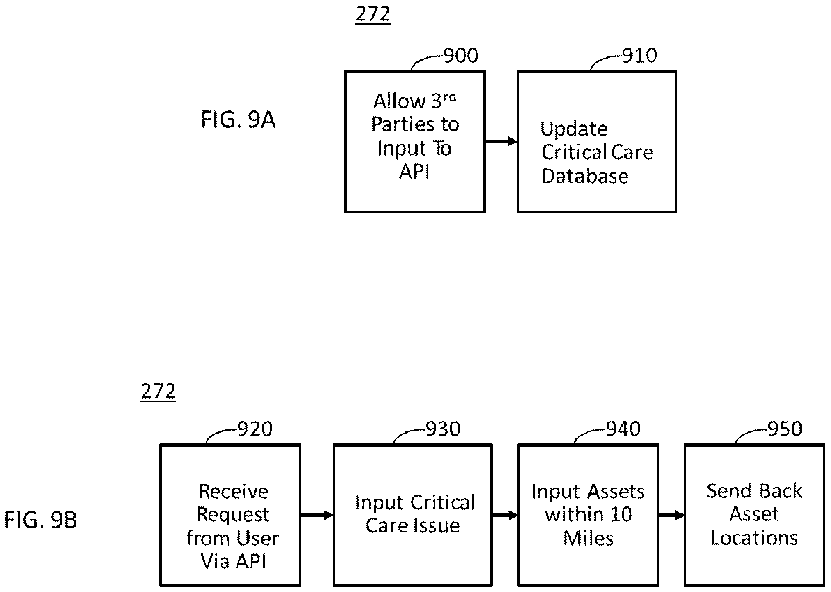

FIGS. 9A-B are flowcharts illustrating exemplary operations of an exemplary critical care software module 272 of the critical care network 270.

FIG. 9A is a flowchart illustrating exemplary third party operations of exemplary critical care software module 272 of the critical care network 270. FIG. 9A particularly illustrates that third parties may be allowed to provide input (*e.g.,* geofence critical care maps 290) to the API 274 (step 900) and that critical care databases 276 found on the critical care network 270 may be updated based on such input (step 910).

FIG. 9B is a flowchart illustrating exemplary user operations of exemplary critical care software module 272 of the critical care network 270. FIG. 9B particularly illustrates that a request may be received from a user 280 via an API 274 of the critical care network 270 (step 920). In particular, the user has provided information regarding the critical care issue/condition of interest (step 930), critical care assets of interest (step 940), and a desired radius for the geofence (*e.g.,* 10 miles) (step 940). Responsive to such information, the critical care database 276 may provide the user with all the locations of all available assets (step 950).

While the flow diagrams in FIG. 9A and FIG. 9B show a particular order of operations performed by certain embodiments of the invention, it should be understood that such order is exemplary (e.g., alternative embodiments can perform the operations in a different order, combine certain operations, overlap certain operations, etc.).

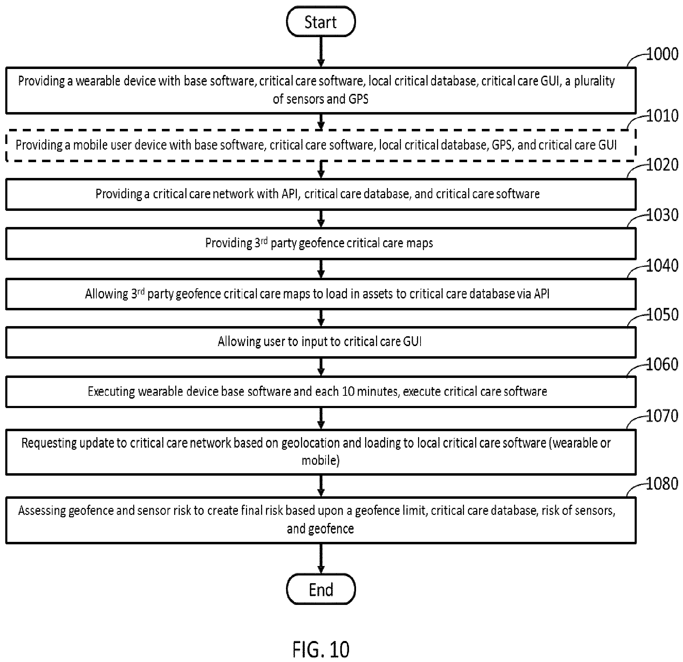

FIG. 10 is a flowchart illustrating an exemplary method for providing a wearable device with critical care capabilities. A wearable device 220 may be provided with certain features (*e.g.,* base software module 234, critical care software module 236, local critical care database in memory 240, critical care GUI 238, sensors 242, and/or GPS module 230) (step 1000). In some embodiments, a user mobile device 250 may be provided with certain features (*e.g.,* base software module 262, critical care software module 266, local critical care database in memory 260, critical care GUI 264, sensors, and/or GPS 266) (step 1010). A server of the critical care network 270 may also be provided with such features as base software module, critical care software module 272, critical care database 276 in memory, and an API 274 (step 1020).

Third parties may be allowed to provide geofence critical care maps 290 for assets of interest (step 1030). Such geofence critical care maps 290 (which may include information regarding asset locations) may be uploaded to the critical care network through the API interface of the network (step 1040).

The user 280 may be able to provide inputs on their respective devices (*e.g.,* via critical care GUI 238 of wearable device 220 and/or critical care GUI 264 of user mobile device 250 and/or a separate online portal interface that interacts with API 274), which may further be sent to the server of the critical care network 270 (step 1050). Such inputs could identify the parameters to be monitored, critical care issues, assets of interest, and/or geofence size. The critical care software and base software on the respective device(s) may be executed and repeated over a regular interview (*e.g.,* ten minutes) (step 1060). The devices may then be able to request updates from the critical care network, so that the critical care database included in the devices could be updated with the information provided from the critical care database found in the critical care network (step 1070).

Based on the information from the critical care database, a risk assessment is provided to the user to view. The risk assessment is based on a number of factors and analyses, including critical care issue risks as detected and determined by one or more sensors a number of assets within a predetermined radius from the wearable device and/or an availability of assets within a geofence (step 1080).

While the flow diagram in FIG. 10 shows a particular order of operations performed by certain embodiments of the invention, it should be understood that such order is exemplary (e.g., alternative embodiments can perform the operations in a different order, combine certain operations, overlap certain operations, etc.).

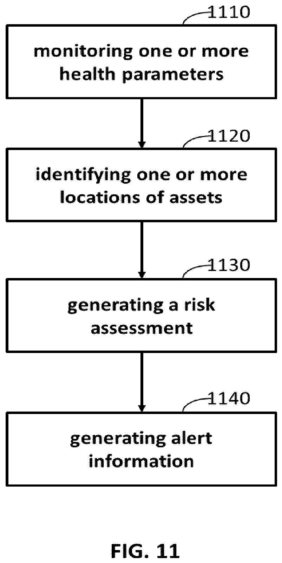

FIG. 11 shows a block diagram of a a few steps of a computer-implemented method for providing critical care using a wearable device according to claim 1. The method includes monitoring one or more health parameters related to a critical care condition of a user of a wearable device via one or more sensors in the wearable device (step 1110) and identifying one or more locations of assets relevant to the critical care condition (step 1120). The method also inlcudes generating a risk assessment based on values of the health parameters (step 1130) and a number of the assets located within a predetermined radius from a current location of the wearable device and generating alert information to the user of the wearable device based on the risk assessment (step 1140). At least some steps of the method are performed by a processor of the wearable device.

In some embodiments, the radius for identifying the assets is determined based on a maximal period of time required for accessing the asset for different types of the critical care condition. The maximal period also depends on a type of a transportation the user can select to reach the asset. Alternatively, the maximal period also depends on a type of a transportation that the user is currently used. The type of the transportation can be determined or detected by one or more sensors in the wearable device, such as accelometer. Additionally or alternatively, the determining the likelihood of availability of required assets and update the risk assessment and/or the maximal period of time to acess the asset based on the likelihood of availability. For example, one embodiment compares the likelihood of availability with a threshold determined based on the type of critical care condition to update the risk assessment.

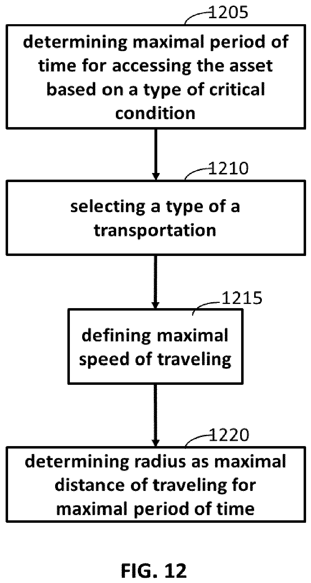

FIG. 12 shows a block diagram of a method for determining the radius for generating a risk assessment according to some embodiments of the invention. The method determines a maximal period of time for accessing the asset based on a type of the critical care condition (step 1205). Such a maximal time can be provided by a doctor and/or can be accessed from the description of the critical care condition.

Next, the user selects a type of a transportation the user is planning to use to access the asset (step 1210). For example, the user can specify the type of transportation as traveling by car using an interface similar to the interface of FIG. 4. The selected type of transportation defines the maximal speed of traveling (step 1215). For example, in urban environment, the maximal speed of traveling by car can be 30 mph. Using the maximal period of time and the maximal speed, the radius is determined as a maximal distance of traveling for the maximal period of time using the selected transportation (step 1220).

Embodiments of the invention also relate to an apparatus for performing the operations herein. Such a computer program is stored in a non-transitory computer readable medium. A machine-readable medium includes any mechanism for storing information in a form readable by a machine (e.g., a computer). For example, a machine-readable (e.g., computer-readable) medium includes a machine (e.g., a computer) readable storage medium (e.g., read only memory ("ROM"), random access memory ("RAM"), magnetic disk storage media, optical storage media, flash memory devices).

The processes or methods depicted in the preceding figures can be performed by processing logic that comprises hardware (e.g. circuitry, dedicated logic, etc.), software (e.g., embodied on a non-transitory computer readable medium), or a combination of both. Although the processes or methods are described above in terms of some sequential operations, it should be appreciated that some of the operations described can be performed in a different order. Moreover, some operations can be performed in parallel rather than sequentially.

The foregoing detailed description of the technology herein has been presented for purposes of illustration and description. It is not intended to be exhaustive or to limit the technology to the precise form disclosed. Many modifications and variations are possible in light of the above teaching. The described embodiments were chosen in order to best explain the principles of the technology and its practical application to thereby enable others skilled in the art to best utilize the technology in various embodiments and with various modifications as are suited to the particular use contemplated. It is intended that the scope of the technology be defined by the claims.

## Claims

1. A computer-implemented method for providing critical care using a wearable device, the method comprising:
monitoring one or more health parameters related to a critical care condition of a user of the wearable device via one or more sensors in the wearable device;
identifying one or more locations of assets relevant to the critical care condition;
generating a risk assessment based on values of the monitored one or more health parameters and a number of the assets located within a radius from a current location of the wearable device;
generating alert information for the user of the wearable device based on the generated risk assessment; and
calling another device and/ or sending an SMS or email message to another device, wherein the calling and/ or the sending is based on the generated risk assessment;
wherein at least one step of the method is performed by a processor of the wearable device.

2. The method of claim 1, wherein the critical care condition can be selected from the group consisting of: heart attack, pneumonia, obesity, diabetes, allergies, injury, and surgery.

3. The method of claim 1, wherein each asset relevant to the critical care condition can be selected from the group consisting of: a defibrillator, a hospital, an insulin supply, an emergency room, a wheelchair, a pair of crutches, an emergency services professional, and an epinephrine autoinjector.

4. The method of claim 1, further comprising:
determining a maximal period of time for accessing the asset based on a type of the critical care condition;
detecting a type of a transportation; and
determining the radius as a maximal distance of traveling for the maximal period of time using the detected transportation.

5. The method of claim 1, further comprising:
determining a likelihood of availability of each identified asset; and
updating the risk assessment based on the likelihood of availability of each identified asset.

6. The method of claim 5, wherein the updating the risk assessment comprises:
comparing the likelihood of availability of each identified asset with a threshold; and
increasing a value of the risk assessment if the likelihood of availability of each identified asset is below the threshold.

7. The method of claim 5, wherein the determining the likelihood of availability of each identified asset comprises:
determining a number of other users near each identified asset, and wherein each of the other users also have a respective critical condition related to the identified assets; and
determining the likelihood of availability of each identified asset based on the number of other users.

8. The method of claim 5, wherein determining the likelihood of availability of each identified asset comprises:
determining a number of other users currently using an identified asset; and
determining the likelihood of availability of each identified asset based on the number of other users.

9. The method of claim 1, wherein the processor of the wearable device is in communication with a user mobile device and a critical care network server.

10. The method of claim 1, wherein generating the alert information to the user of the wearable device based on the risk assessment includes displaying a notification based on the risk assessment on a display screen of the wearable device, wherein the notification can be selected from the group consisting of: a message, a graphic, and a video,
wherein the notification is generated by a critical care network server connected to the wearable device.

11. The method of claim 1, wherein generating the alert information to the user of the wearable device based on the risk assessment can be selected from the group consisting of: playing a sound, generating a vibration, and generating a small electric shock, wherein the generating alert information depends on a value of the risk assessment.

12. The method of claim 1, further comprising:
storing critical care information in a memory of the wearable device, the critical care information being associated with the critical care condition of the user of the wearable device.

13. A system comprising:
one or more critical care network servers, each server providing information regarding locations of assets, each asset designated as being relevant to one or more critical care conditions; and
a wearable device comprising:
a memory configured to store information regarding a critical care condition associated with a user;
one or more sensors configured to monitor one or more health parameters related to the critical care condition associated with the user;
a processor configured for:
identifying locations of assets determined to be relevant to the condition of interest to the user, wherein the locations are identified as being within a predetermined radius of a current location of the user;
generating a risk assessment based on the monitored parameters and a number of the assets located within a radius from a current location of the wearable device;
generating alert information to the user of the wearable device based on the risk assessment;
calling another device and/ or sending an SMS or email message to another device, wherein the calling and/ or the sending is based on the generated risk assessment; and
a display screen configured to display the alert information.

14. The system of claim 13, wherein the alert information displayed by the display screen of the wearable device is accompanied by a secondary alert, the secondary alert can be selected from the group consisting of: playing a sound, generating a vibration, and generating a small electric shock.

15. A non-transitory computer-readable storage medium, having embodied thereon a program executable by a processor to perform a method for providing on-demand wireless services, the method comprising:
storing critical care information in a memory of the wearable device, the critical care information associated with a critical care condition of a user of the wearable device;
monitoring one or more health parameters related to the critical care condition via one or more sensors in the wearable device;
identifying one or more assets within a predetermined radius of a current location of the user, each asset is relevant to the critical care condition;
generating a risk assessment based on the one or more health parameters and a number of the assets located within a radius from a current location of the wearable device;
generating alert information to a user of the wearable device based on the risk assessment; and
calling another device and/ or sending an SMS or email message to another device, wherein the calling and/ or the sending is based on the generated risk assessment.

## Patentansprüche

1. Eine auf einem Computer auszuführende Methode für die Intensivpflege mithilfe eines tragbaren Geräts, wobei die Methode folgende Schritte umfasst:
Überwachen mindestens eines Gesundheitsparameters,
der mit dem Intensivpflegezustand des Benutzers des tragbaren Geräts zusammenhängt, mithilfe mindestens einer der Sensoren des tragbaren Geräts;
Ermitteln mindestens eines Standorts der für den Intensivpflegezustand relevanten Maßnahmen;
Generieren einer Risikobewertung anhand der Werte des mindestens einen überwachten Gesundheitsparameters sowie einiger der Maßnahmen, die sich innerhalb eines Radius vom aktuellen Standort des tragbaren Geräts befinden;
Generieren von Alarminformationen für den Benutzer des tragbaren Geräts beruhend auf der generierten Risikobewertung; und
Anrufen eines anderen Geräts und/oder Senden einer SMS- oder E-Mail-Nachricht an ein anderes Gerät,
wobei das Anrufen und/oder Senden auf der generierten Risikobewertung beruht;
wobei mindestens ein Schritt der Methode vom Prozessor des tragbaren Geräts durchgeführt wird.

2. Die Methode gemäß Anspruch 1, wobei der Intensivpflegezustand aus folgender Gruppe ausgewählt werden kann: Herzinfarkt, Lungenentzündung, Fettleibigkeit, Diabetes, Allergie, Verletzung und Operation.

3. Die Methode gemäß Anspruch 1, wobei die einzelnen für den Intensivpflegezustand relevanten Maßnahmen aus folgender Gruppe ausgewählt werden können:
ein Defibrillator, ein Krankenhaus, ein Insulinvorrat, eine Notaufnahme,
ein Rollstuhl, ein
Paar Krücken, ein Notdienstmitarbeiter und ein Epinephrin-Autoinjektor.

4. Die Methode gemäß Anspruch 1, die zudem folgende Schritte umfasst:
Ermitteln der maximalen Zeitspanne für den Zugriff auf die Maßnahme beruhend auf dem Typ des Intensivpflegezustands;
Ermitteln der Beförderungsart; und
Ermitteln des Radius als maximale Fahrstrecke des ermittelten Transportmittels in der maximalen Zeitspanne.

5. Die Methode gemäß Anspruch 1, die zudem folgende Schritte umfasst:
Ermitteln der Verfügbarkeitswahrscheinlichkeit der einzelnen ermittelten Maßnahmen; und
Aktualisieren der Risikobewertung beruhend auf der Verfügbarkeitswahrscheinlichkeit der einzelnen ermittelten Maßnahmen.

6. Die Methode gemäß Anspruch 5, wobei das Aktualisieren der Risikobewertung folgende Schritte umfasst:
Vergleichen der Verfügbarkeitswahrscheinlichkeit der einzelnen ermittelten Maßnahmen mit einem Schwellenwert; und
Erhöhen des Werts der Risikobewertung, wenn die Verfügbarkeitswahrscheinlichkeit der einzelnen ermittelten Maßnahmen unter dem Schwellenwert liegt.

7. Die Methode gemäß Anspruch 5, wobei das Ermitteln der Verfügbarkeitswahrscheinlichkeit der einzelnen ermittelten Maßnahmen folgende Schritte umfasst:
Ermitteln der Anzahl anderer Benutzer in der Nähe der einzelnen ermittelten Maßnahmen, und wobei die einzelnen weiteren Benutzer jeweils ebenfalls einen entsprechenden kritischen Zustand in Bezug auf die ermittelten Maßnahmen aufweisen; und
Ermitteln der Verfügbarkeitswahrscheinlichkeit der einzelnen ermittelten Maßnahmen beruhend auf der Anzahl anderer Benutzer.

8. Die Methode gemäß Anspruch 5, wobei das Ermitteln der Verfügbarkeitswahrscheinlichkeit der einzelnen ermittelten Maßnahmen folgende Schritte umfasst:
Ermitteln der Anzahl anderer Benutzer, die derzeit eine der einzelnen ermittelten Maßnahmen nutzen; und
Ermitteln der Verfügbarkeitswahrscheinlichkeit der einzelnen ermittelten Maßnahmen beruhend auf der Anzahl anderer Benutzer.

9. Die Methode gemäß Anspruch 1, wobei der Prozessor des tragbaren Geräts mit einem mobilen Gerät des Benutzers und einem Intensivpflege-Netzwerkserver kommuniziert.

10. Die Methode gemäß Anspruch 1, wobei das auf der Risikobewertung beruhende Generieren der Alarminformationen für den Benutzer des tragbaren Geräts das Anzeigen einer auf der Risikobewertung beruhenden Benachrichtigung auf einem Anzeigebildschirm des tragbaren Geräts umfasst, wobei die Benachrichtigung aus folgender Gruppe ausgewählt werden kann: eine Meldung, eine Grafik und ein Video,
wobei die Benachrichtigung von einem Intensivpflege-Netzwerkserver generiert wird, der mit dem tragbaren Gerät verbunden ist.

11. Die Methode gemäß Anspruch 1, wobei das auf der Risikobewertung beruhende Generieren der Alarminformationen für den Benutzer des tragbaren Geräts aus folgender Gruppe ausgewählt werden kann: Wiedergeben eines Tons, Erzeugen einer Vibration und Erzeugen eines kleinen elektrischen Schocks, wobei das Erzeugen der Alarminformationen vom Wert der Risikobewertung abhängt.

12. Die Methode gemäß Anspruch 1, die zudem folgenden Schritt umfasst:
Speichern von Intensivpflegedaten auf einem Speicher des tragbaren Geräts, wobei die Intensivpflegedaten mit dem Intensivpflegezustand des Benutzers des tragbaren Geräts verknüpft sind.

13. Ein System, das Folgendes umfasst:
Mindestens einen Intensivpflege-Netzwerkserver, wobei die einzelnen Server Daten über die Standorte der Maßnahmen bereitstellen, und wobei die einzelnen Maßnahmen für mindestens einen Intensivpflegezustand als relevant gekennzeichnet sind; und
Ein tragbares Gerät, das Folgendes umfasst:
einen Speicher, in dem Daten über den einem Benutzer zugeordneten Intensivpflegezustand gespeichert werden;
mindestens einen Sensor, der mindestens einen mit dem Intensivpflegezustand und dem Benutzer verknüpften Gesundheitsparameter überwacht;
einen Prozessor, der folgende Schritte durchführt:
Ermitteln der Standorte der für den Zustand des Benutzers relevanten Maßnahmen, wobei die Standorte als innerhalb eines vorab festgelegten Radius vom aktuellen Standort des Benutzers liegend ermittelt werden;
Generieren einer Risikobewertung anhand der überwachten Parameter sowie einiger der Maßnahmen,
die sich innerhalb eines Radius vom aktuellen Standort des tragbaren Geräts befinden;
Generieren von Alarminformationen für den Benutzer des tragbaren Geräts beruhend auf der Risikobewertung;
Anrufen eines anderen Geräts und/oder Senden einer SMS- oder E-Mail-Nachricht an ein anderes Gerät,
wobei das Anrufen und/oder Senden auf der generierten Risikobewertung beruht; und
einen Anzeigebildschirm zum Anzeigen der Alarminformationen.

14. Das System gemäß Anspruch 13, wobei die auf dem Anzeigebildschirm des tragbaren Geräts angezeigten Alarminformationen von einem sekundären Alarm begleitet werden, und wobei der sekundäre Alarm aus folgender Gruppe ausgewählt werden kann: Wiedergeben eines Tons, Erzeugen einer Vibration und Erzeugen eines kleinen elektrischen Schocks.

15. Ein nicht flüchtiges, von einem Computer lesbares Speichermedium, auf dem sich ein Programm befindet, das von einem Prozessor ausgeführt werden kann, um eine Methode zum Bereitstellen drahtloser On-Demand-Dienste umzusetzen, wobei die Methode folgende Schritte umfasst:
Speichern von Intensivpflegedaten auf einem Speicher des tragbaren Geräts, wobei die Intensivpflegedaten mit dem Intensivpflegezustand des Benutzers des tragbaren Geräts verknüpft sind;
Überwachen mindestens eines mit dem Intensivpflegezustand zusammenhängenden Gesundheitsparameters mithilfe mindestens einer der Sensoren des tragbaren Geräts;
Ermitteln mindestens einer Maßnahme innerhalb eines vorab festgelegten Radius des aktuellen Standorts des Benutzers, wobei die einzelnen Maßnahmen jeweils für den Intensivpflegezustand relevant sind;
Generieren einer Risikobewertung anhand des mindestens einen Gesundheitsparameters sowie einiger der Maßnahmen, die sich innerhalb eines Radius vom aktuellen Standort des tragbaren Geräts befinden;
Generieren von Alarminformationen für den Benutzer des tragbaren Geräts beruhend auf der Risikobewertung; und
Anrufen eines anderen Geräts und/oder Senden einer SMS- oder E-Mail-Nachricht an ein anderes Gerät,
wobei das Anrufen und/oder Senden auf der generierten Risikobewertung beruht.

## Revendications

1. Procédé mis en œuvre par ordinateur permettant de fournir des soins critiques à l'aide d'un dispositif portable, ledit procédé comprenant :
la surveillance d'au moins un paramètre de santé lié à un état de soins critiques d'un utilisateur du dispositif portable par l'intermédiaire d'au moins un capteur dans le dispositif portable ;
l'identification d'au moins un emplacement d'actifs pertinents pour l'état de soins critiques ;
la génération d'une évaluation du risque en fonction des valeurs de l'au moins un paramètre de santé surveillé et d'un nombre d'actifs situés dans un rayon à partir d'un emplacement actuel du dispositif portable ;
la génération des informations d'alerte pour l'utilisateur du dispositif portable en fonction de l'évaluation du risque générée ; et
l'appel d'un autre dispositif et/ou l'envoi d'un SMS ou d'un message électronique à un autre dispositif,
dans lequel l'appel et/ou l'envoi est basé sur l'évaluation du risque générée ;
dans lequel au moins une étape du procédé est effectuée par un processeur du dispositif portable.

2. Procédé selon la revendication 1, dans lequel l'état de soins critiques peut être sélectionné dans le groupe constitué par : une crise cardiaque, une pneumonie, une obésité, un diabète, des allergies, une blessure et un acte chirurgical.

3. Procédé selon la revendication 1, dans lequel chaque actif pertinent pour l'état de soins critiques peut être sélectionné dans le groupe constitué par :
un défibrillateur, un hôpital, une alimentation en insuline, une salle d'urgence,
un fauteuil roulant, une
paire de béquilles, un professionnel des services de secours et un injecteur automatique d'épinéphrine.

4. Procédé selon la revendication 1, comprenant en outre :
la détermination d'une période de temps maximale pour accéder à l'actif en fonction d'un type d'état de soins critiques ;
la détection d'un type de transport ; et
la détermination du rayon en tant que distance maximale de déplacement pendant la période de temps maximale à l'aide du transport détecté.

5. Procédé selon la revendication 1, comprenant en outre :
la détermination d'une probabilité de disponibilité de chaque actif identifié ; et
la mise à jour de l'évaluation du risque en fonction de la probabilité de disponibilité de chaque actif identifié.

6. Procédé selon la revendication 5, dans lequel la mise à jour de l'évaluation du risque comprend :
la comparaison de la probabilité de disponibilité de chaque actif identifié avec un seuil ; et
l'augmentation de la valeur de l'évaluation du risque lorsque la probabilité de disponibilité de chaque actif identifié est inférieure au seuil.

7. Procédé selon la revendication 5, dans lequel la détermination de la probabilité de disponibilité de chaque actif identifié comprend :
la détermination d'un nombre d'autres utilisateurs à proximité de chaque actif identifié, et dans lequel chacun des autres utilisateurs présente également un état critique respective liée aux actifs identifiés ;
et
la détermination de la probabilité de disponibilité de chaque actif identifié en fonction du nombre d'autres utilisateurs.

8. Procédé selon la revendication 5, dans lequel la détermination de la probabilité de disponibilité de chaque actif identifié comprend :
la détermination d'un nombre d'autres utilisateurs utilisant actuellement un actif identifié ; et
la détermination de la probabilité de disponibilité de chaque actif identifié en fonction du nombre d'autres utilisateurs.

9. Procédé selon la revendication 1, dans lequel le processeur du dispositif portable est en communication avec un dispositif mobile utilisateur et un serveur de réseau de soins critiques.

10. Procédé selon la revendication 1, dans lequel la génération des informations d'alerte à l'utilisateur du dispositif portable en fonction de l'évaluation du risque comprend l'affichage d'une notification en fonction de l'évaluation du risque sur un écran d'affichage du dispositif portable, dans lequel la notification peut être sélectionnée dans le groupe constitué par : un message, un graphique et une vidéo,
dans lequel la notification est générée par un serveur de réseau de soins critiques connecté au dispositif portable.

11. Procédé selon la revendication 1, dans lequel la génération des informations d'alerte à l'utilisateur du dispositif portable en fonction de l'évaluation du risque peut être sélectionnée dans le groupe constitué par : la reproduction d'un son, la génération d'une vibration et la génération d'un petit choc électrique, dans lequel la génération des informations d'alerte dépend d'une valeur de l'évaluation du risque.

12. Procédé selon la revendication 1, comprenant en outre :
la mémorisation des informations de soins critiques dans une mémoire du dispositif portable, les informations de soins critiques étant associées à l'état de soins critiques de l'utilisateur du dispositif portable.

13. Système comprenant :
au moins un serveur de réseau de soins critiques,
chaque serveur fournissant des informations concernant les emplacements des actifs, chaque actif étant désigné comme étant pertinent pour au moins un état de soins critiques ; et
un dispositif portable comprenant :
une mémoire configurée pour mémoriser des informations concernant un état de soins critiques associé à un utilisateur ;
au moins un capteur configuré pour surveiller au moins un paramètre de santé lié à l'état de soins critiques associée à l'utilisateur ;
un processeur configuré :
pour identifier des emplacements d'actifs déterminés comme pertinents par rapport à l'état d'intérêt de l'utilisateur, dans lequel les emplacements sont identifiés comme situés dans un rayon prédéterminé d'un emplacement actuel de l'utilisateur ;
pour générer une évaluation du risque en fonction des paramètres surveillés et du nombre d'actifs situés dans un rayon à partir d'un emplacement actuel du dispositif portable ;
pour générer des informations d'alerte à l'utilisateur du dispositif portable en fonction de l'évaluation du risque ;
pour appeler un autre dispositif et/ou pour envoyer un SMS ou un message électronique à un autre dispositif, l'appel et/ou l'envoi étant basés sur l'évaluation du risque générée ; et
un écran d'affichage conçu pour afficher les informations d'alerte.

14. Système selon la revendication 13, dans lequel les informations d'alerte affichées par l'écran d'affichage du dispositif portable sont accompagnées d'une alerte secondaire, l'alerte secondaire pouvant être sélectionnée dans le groupe constitué par : la reproduction d'un son, la génération d'une vibration et la génération d'un petit choc électrique.

15. Support d'enregistrement non transitoire lisible par ordinateur, sur lequel un programme, exécutable par un processeur pour mettre en œuvre un procédé pour fournir des services sans fil à la demande, est incorporé, ledit procédé comprenant :
la mémorisation des informations de soins critiques dans une mémoire du dispositif portable, les informations de soins critiques étant associées à un état de soins critiques d'un utilisateur du dispositif portable ;
la surveillance d'au moins un paramètre de santé lié à l'état de soins critiques par l'intermédiaire d'au moins un capteur dans le dispositif portable ;
l'identification de l'au moins un actif dans un rayon prédéterminé de l'emplacement actuel de l'utilisateur, chaque actif étant pertinent pour l'état de soins critiques ;
la génération d'une évaluation du risque en fonction de l'au moins un paramètre de santé et d'un nombre d'actifs situés dans un rayon à partir d'un emplacement actuel du dispositif portable ;
la génération des informations d'alerte à un utilisateur du dispositif portable en fonction de l'évaluation du risque ; et
l'appel d'un autre dispositif et/ou l'envoi d'un SMS ou d'un message électronique à un autre dispositif, l'appel et/ou l'envoi étant basés sur l'évaluation du risque générée.
